# EUROPEAN PATENT APPLICATION

(11) **EP 2 620 128 A1**
(43) Date of publication of application: **31.07.2013**
(21) Application number: 11826737.6
(22) Date of filing: 08.09.2011
(51) Int. Cl.: A61F 2/82, A61B 17/00, A61L 31/00, A61M 25/00

(54) **BIOLOGICAL ADHESIVE SHEET AND DEVICE FOR BONDING SHEET**

(30) Priority: 22.09.2010 JP 2010212797; 22.09.2010 JP 2010212795
(71) Applicant: Terumo Kabushiki Kaisha, Tokyo 151-0072 (JP)
(72) Inventor: ISHII, Naoki, Ashigarakami-gun Kanagawa 259-0151 (JP); MORISHITA, Keitaro, Fujinomiya-shi Shizuoka 418-0015 (JP); SOBA, Ryoichi, Fujinomiya-shi Shizuoka 418-0015 (JP)
(74) Representative: Dossmann, Gérard
(86) International application number: PCT/JP2011/070493
(87) International publication number: WO 2012/039291

(57) **Abstract**

[Object]

To provide a biological adhesive sheet which is reduced in the influence on a living organism and thus has improved safety.

[Solution]

A biological adhesive sheet (10) which has: a substrate (12) that is formed of a biocompatible material; and a plurality of projections (13) that are formed of a biocompatible material and configured so as to protrude from the surface of the substrate (12). The biological adhesive sheet (10) is bonded to living tissues by the van der Waals force by having the plurality of projections (13) in contact with the living tissues.

## Description

### Technical Field

The present invention relates to a sheet to be bonded (adhered) to a living tissue and a device for bonding the sheet to the living tissue. Particularly, the invention relates to a medical sheet which can be bonded to the inside of a body lumen and a device for delivering and bonding the sheet to the inside of the body lumen.

### Background Art

As a therapeutic device for use to the inside of a body lumen, there have been known, for example, tubular stents which are set indwelling in blood vessels (see, for example, Patent Document 1). A stent is ordinarily used in the following manner. First, a blood vessel at the patient's leg or arm is minutely incised, and an introducer sheath (introducer) is set there. While a guide wire is precedingly advanced through the lumen of the introducer sheath, a stent in a radially contracted state is inserted into the blood vessel by a balloon catheter or the like. Thereafter, the stent is expanded in a target position by the balloon or the like, to be set indwelling in the blood vessel. By being set indwelling in the blood vessel, the stent functions to support a stenosed part from the inside or to obstruct an entrance to an aneurysm.

Such a stent is commonly formed in a tubular shape. Even in the case where only a part in the circumferential direction of a blood vessel is troubled, therefore, the stent makes contact with the whole part ranging over 360 degrees along the circumferential direction of the blood vessel, and is set indwelling in a wide range inclusive of healthy parts of the blood vessel.

Besides, once set indwelling, the stent continues to be present in the blood vessel, without being taken out. When the stent, which is a foreign matter, is present in a blood vessel, however, a thrombus may be generated due to the stent. Therefore, the patient must continue to be medicated with an antiplatelet agent as an antithrombogenic treatment. Although there is present a drug-eluting stent having a drug contained in a stent, the period over which the drug is being eluted is limited.

### Prior Art Document

### Patent Document

Patent Document 1: Japanese Patent Laid-open No. 2009-131672

### Summary of Invention

### Technical Problem

The present invention has been made in order to solve the above-mentioned problems. Accordingly, it is an object of the present invention to provide a biological adhesive sheet and a device for bonding (adhering) the sheet which are capable of reducing influence thereof on a living organism and enhancing safety.

### Technical Solution

In order to attain the above object, according to the present invention, there is provided a biological adhesive sheet including: a substrate formed of a biocompatible material; and a plurality of projections which are formed of a biocompatible material and are projected from a surface of the substrate, wherein the biological adhesive sheet is bonded to a living tissue by van der Waals forces by having the plurality of projections in contact with the living tissue.

### Advantageous Effects

The biological adhesive sheet according to the present invention is bonded to a living tissue by the van der Waals force by putting the plurality of projections in contact with the living tissue. Therefore, there is no need for other configuration for holding the bonded state of the biological adhesive sheet, so that the influence on the living organism can be reduced and safety can be enhanced. Especially, in a therapy of the inside of a body lumen where a stent is ordinarily needed, the influence on healthy parts of the tube (vessel) can be reduced. Specifically, when a stent is used in a lumen, there arises the problem that even if only a part in the circumferential direction of the tube (vessel) is troubled, the stent makes contact with the whole body ranging over 360 degrees in the circumferential direction of the tube and the stent is left indwelling in a wide range inclusive of the healthy parts of the tube. When the biological adhesive sheet is used, on the other hand, the sheet can be bonded by the van der Waals force, without utilizing any radial forces as in the case of a stent, so that only the troubled part of the tube can be covered and thereby treated. Thus, the influence on the healthy parts of the tube (vessel) can be minimized.

Where the projections are formed on the surface of the substrate in a density of one or more projections per 1 µm² and have a length of 1 to 500 µm and a maximum outside diameter of 5 nm to 1 µm, the biological adhesive sheet can exhibit a good adhesive force to a living tissue.

Where the substrate and the projections are each formed of a biodegradable polymer, the bonded biological adhesive sheet disappears through decomposition with the lapse of time, whereby the period of medication with an antiplatelet agent can be shortened.

Where the substrate and the projections are each formed of biodegradable polymer such that the substrate disappears through decomposition earlier than the projections, the substrate will disappears through decomposition before the adhesive force owing to the projections is lost. Accordingly, the substrate can be restrained from peeling.

Where the biodegradable polymer is one or more selected from the group consisting of polylactic acid, polylactic acid stereo complex, polyglycolic acid, a copolymer of polylactic acid and polyglycolic acid, polyhydroxybutyric acid, polymalic acid, poly-α-amino acid, collagen, laminin, heparan sulfate, fibronectin, vitronectin, chondroitin sulfate, hyaluronic acid, polycaprolactone, and polyamino acid, the biological adhesive sheet is decomposed favorably after bonded to a living tissue.

Where the substrate is a porous body, it is possible to promote the regeneration of a living tissue to which the biological adhesive sheet is bonded, and to permit the biological adhesive sheet to be speedily covered with the living tissue.

Where at least one of the substrate and the projections contains a biologically active agent, it is possible to treat a living tissue to which the biological adhesive sheet is bonded, or to restrain obstruction or the like from occurring at an application site in the case where the biological adhesive sheet is disposed to the inside of a body lumen.

Where the substrate is a tubular base (or tubular body) and the projections are formed to project from an outer circumferential surface of the base and are formed in a tubular shape, the biological adhesive sheet can be set indwelling safely to the inside of a body lumen, for example, a blood vessel by the van der Waals force while reducing the influence on the living organism.

Where the substrate has a plurality of slits, the biological adhesive sheet can be bonded while enlarging the substrate according to a living organism to which the biological adhesive sheet is applied. Especially in the case where the substrate is a tubular base (or tubular body), the biological adhesive sheet can be bonded while enlarging the base according to the inside diameter of a body lumen to which the biological adhesive sheet is applied.

A device for bonding a sheet may include: a tube which can be inserted into a living organism; a tubular holding section which is disposed at a distal portion of the tube, is cable of being expanded and contracted elastically, and is capable of holding on an outer circumferential surface thereof a biological adhesive sheet to be bonded to a living tissue; an outer sheath which contracts the holding section by covering the outer circumference of the holding section, and permits releases and expansion of the holding section by coming away from the holding section along an axial direction; and a balloon which is disposed at a distal portion of the tube, and is capable of being inflated and deflated. Where the device for bonding a sheet has the just-mentioned configuration, the holding section with the biological adhesive sheet held on the outer circumferential surface thereof is contained in a contracted state in the outer sheath, so that the biological adhesive sheet can be easily delivered to a target position. Furthermore, with the outer sheath moved in the axial direction, the holding section is permitted to expand by its self-expanding function, so that the biological adhesive sheet held on the outer circumferential surface of the holding section can be easily bonded to a living organism by an operation conducted on the proximal side. In addition, since the balloon is provided, the biological adhesive sheet can be bonded to a living organism more assuredly by an operation conducted on the proximal side.

Where the balloon is capable of being inflated and deflated inside the holding section, the biological adhesive sheet can be assuredly bonded to a living tissue by pressing the biological adhesive sheet by the balloon in the condition where the biological adhesive sheet is tentatively fixed to the living tissue by the holding section.

### Brief Description of Drawings

[FIG. 1]

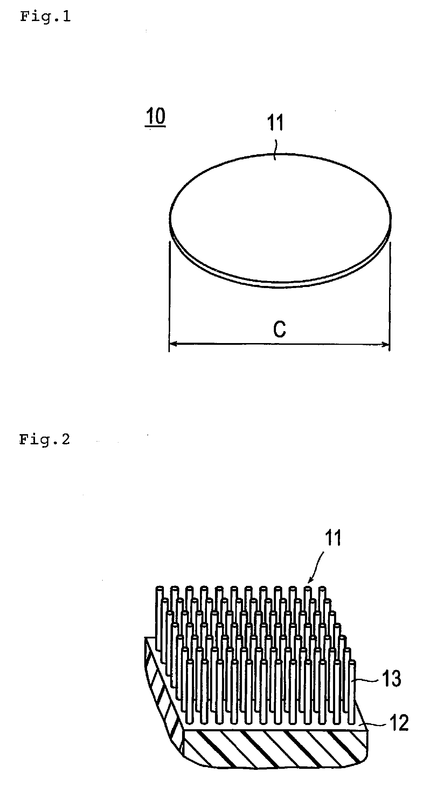

   FIG. 1 is a perspective view showing a biological adhesive sheet according to a first embodiment.
[FIG. 2]
   FIG. 2 is a partly enlarged perspective view showing a part of an adhesion surface of the biological adhesive sheet according to the first embodiment.
[FIG. 3]

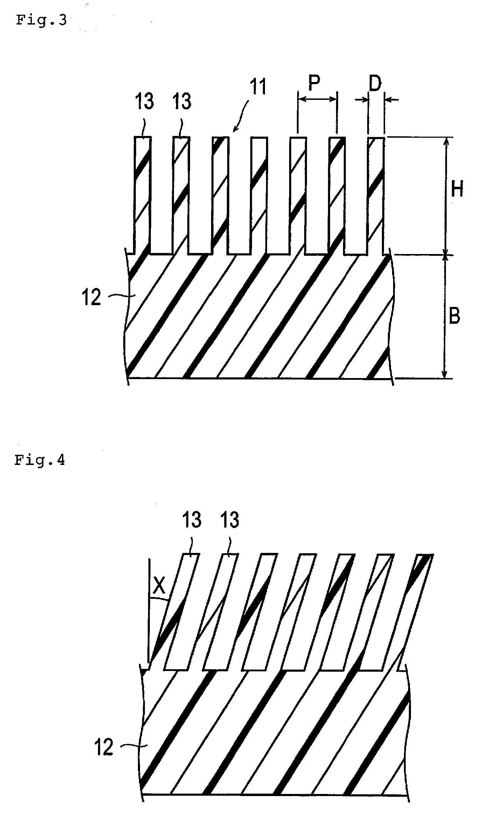

   FIG. 3 is a partly enlarged sectional view showing a part of the adhesion surface of the biological adhesive sheet according to the first embodiment.
[FIG. 4]
   FIG. 4 is a partly enlarged sectional view of the adhesion surface, showing another example of the biological adhesive sheet according to the first embodiment.
[FIG. 5]

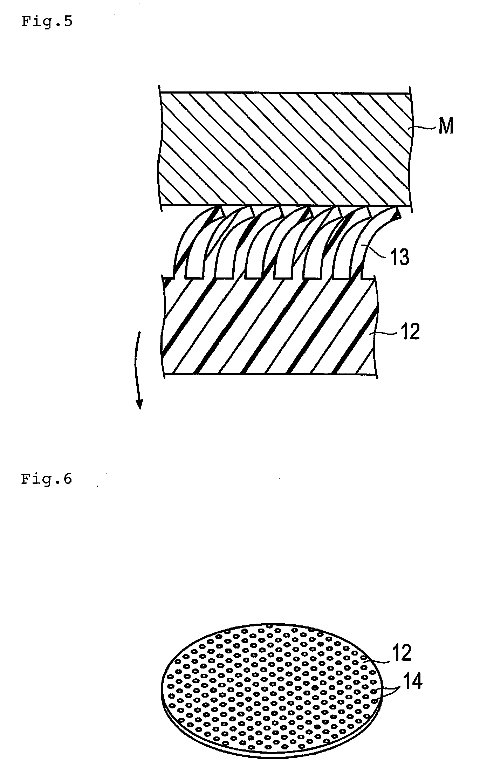

   FIG. 5 is a partly enlarged sectional view showing a mode in which the biological adhesive sheet according to the first embodiment is bonded.
[FIG. 6]
   FIG. 6 is a perspective view showing a further example of the biological adhesive sheet according to the first embodiment.
[FIG. 7]

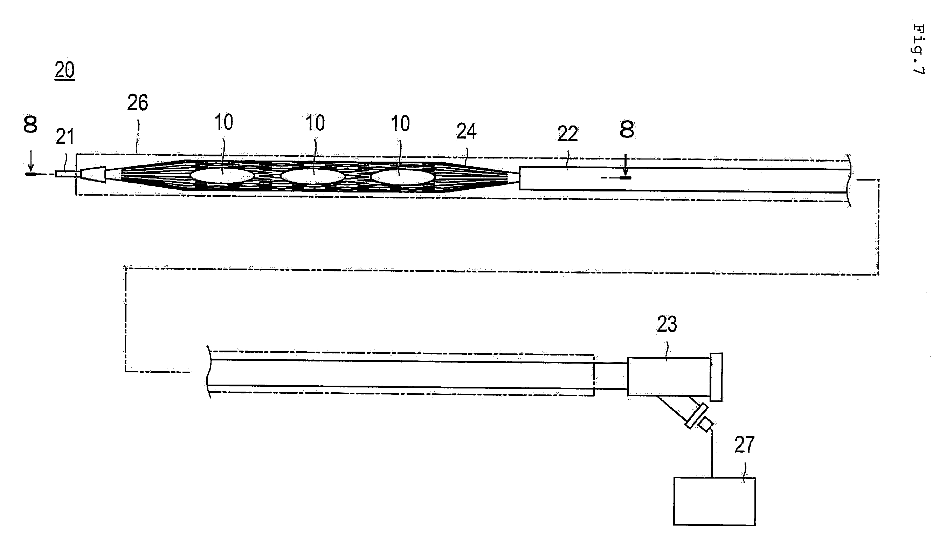

   FIG. 7 is a plan view showing a device for bonding a sheet.
[FIG. 8]

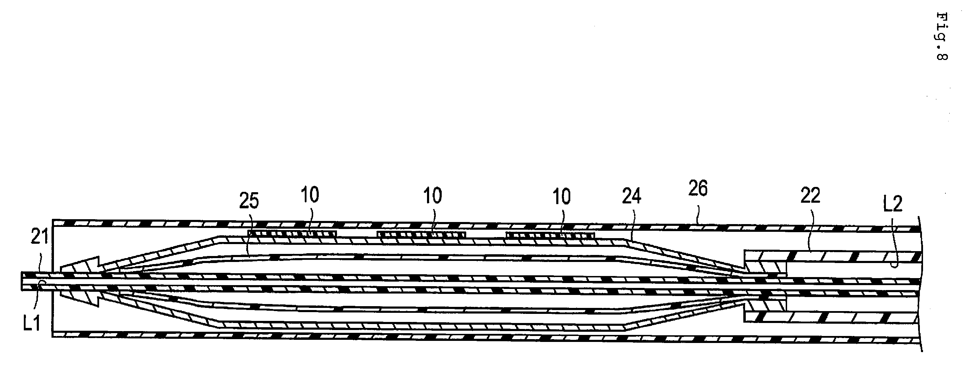

   FIG. 8 is a sectional view taken along line 8-8 of FIG. 7.
[FIG. 9]

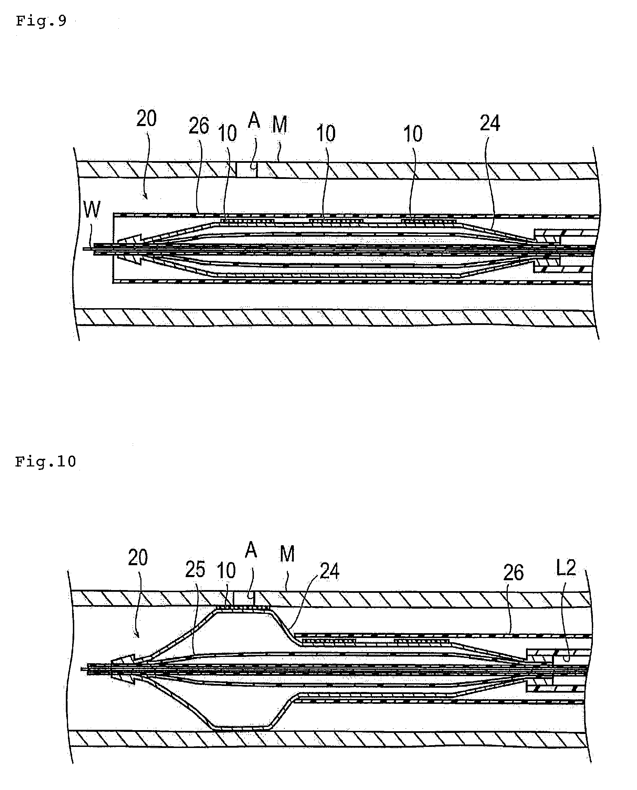

   FIG. 9 is a sectional view showing the condition where the device for bonding a sheet is inserted in a blood vessel.
[FIG. 10]
   FIG. 10 is a sectional view showing the condition where the biological adhesive sheet is tentatively fixed to a lesion by a holding section of the device for bonding a sheet.
[FIG. 11]

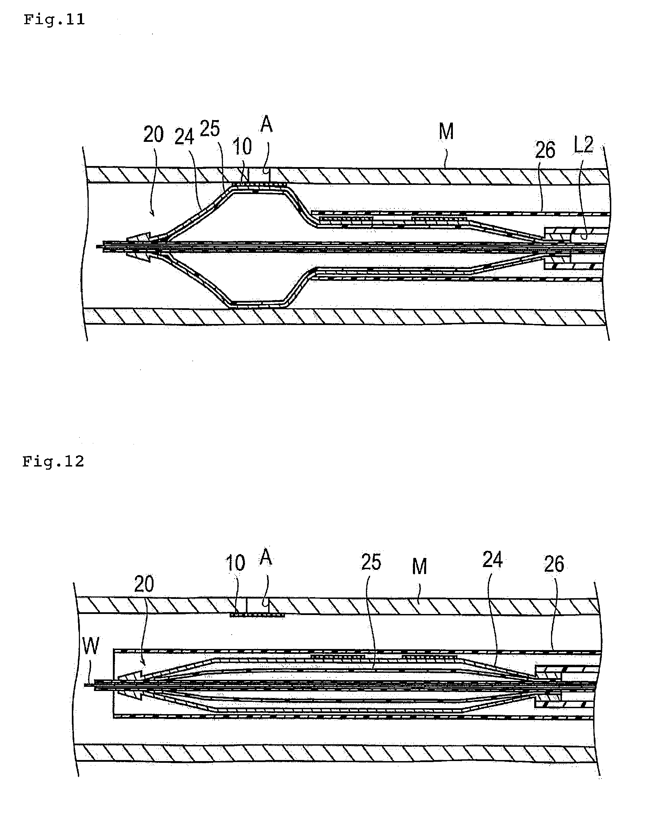

   FIG. 11 is a sectional view showing the condition where the biological adhesive sheet is bonded to the lesion by a balloon of the device for bonding a sheet.
[FIG. 12]
   FIG. 12 is a sectional view showing the condition where the holding section of the device for bonding a sheet is contracted.
[FIG. 13]

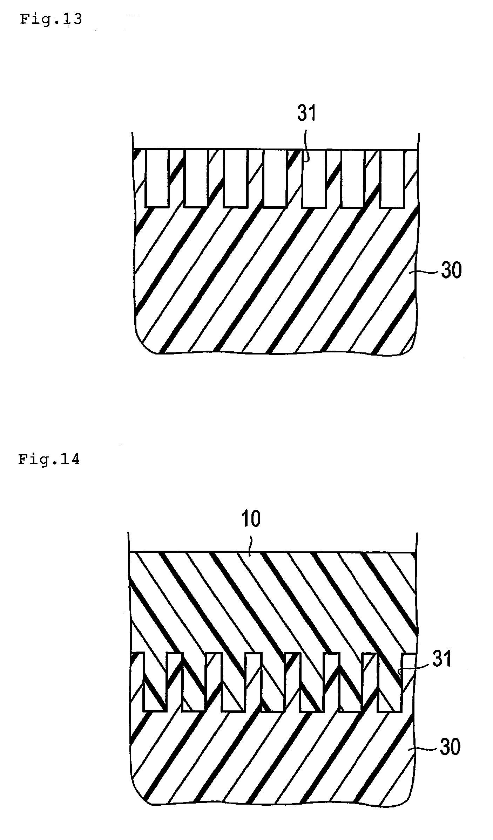

   FIG. 13 is a partly enlarged sectional view showing a mold for producing the biological adhesive sheet.
[FIG. 14]
   FIG. 14 is a partly enlarged sectional view showing the condition where a material for forming the biological adhesive sheet is poured into (onto) the mold.
[FIG. 15]

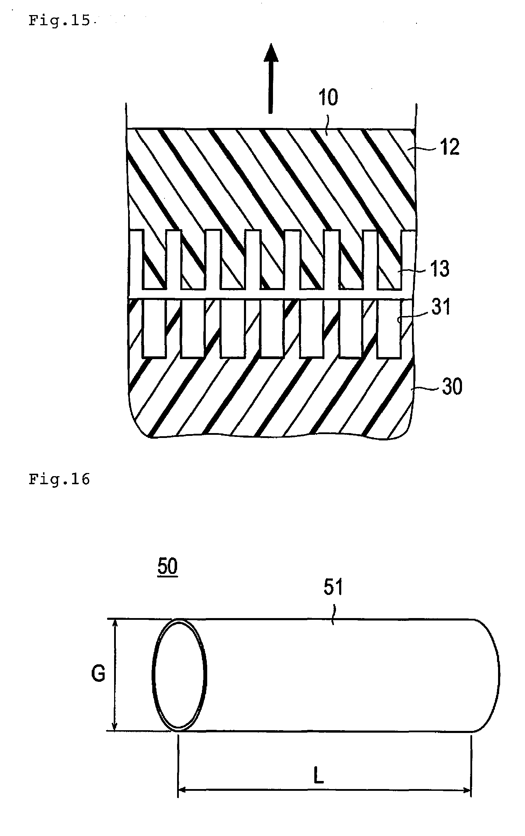

   FIG. 15 is a partly enlarged sectional view showing a mode in which the biological adhesive sheet is detached from the mold.
[FIG. 16]
   FIG. 16 is a perspective view showing a biological adhesive tube according to a second embodiment.
[FIG. 17]

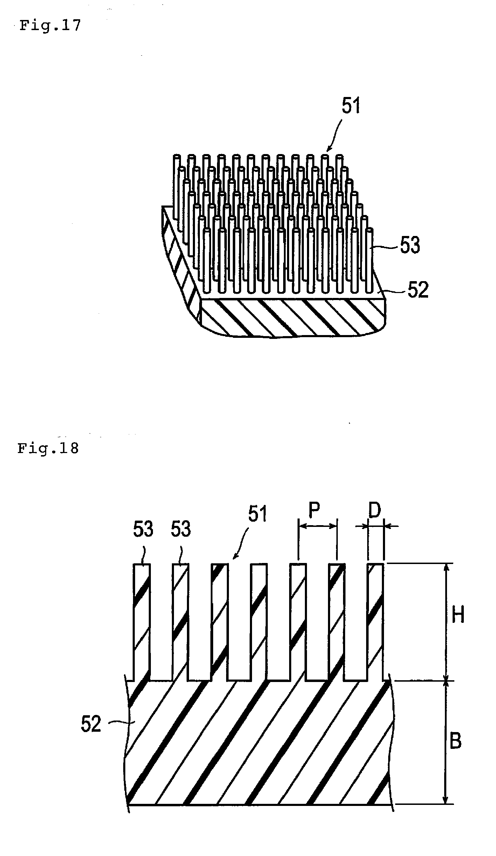

   FIG. 17 is a partly enlarged perspective view showing a part of an adhesion surface of the biological adhesive tube according to the second embodiment.
[FIG. 18]
   FIG. 18 is a partly enlarged sectional view showing a part of the adhesion surface of the biological adhesive tube according to the second embodiment.
[FIG. 19]

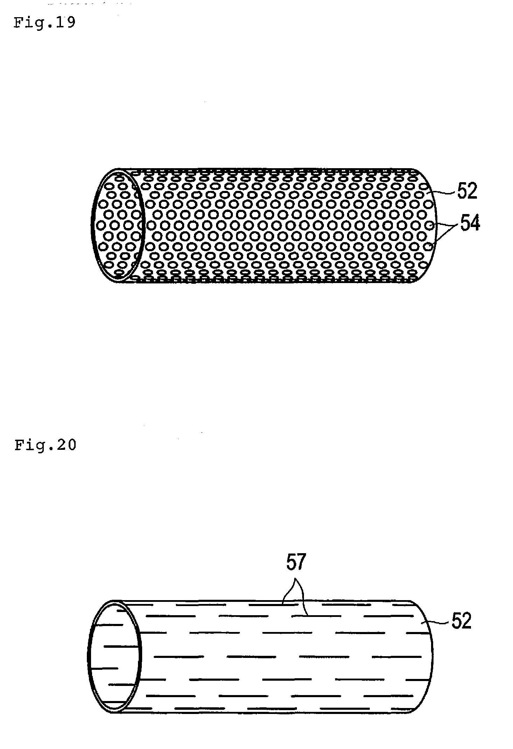

   FIG. 19 is a perspective view showing another example of the biological adhesive tube according to the second embodiment.
[FIG. 20]
   FIG. 20 is a perspective view showing a further example of the biological adhesive tube according to the second embodiment.
[FIG. 21]

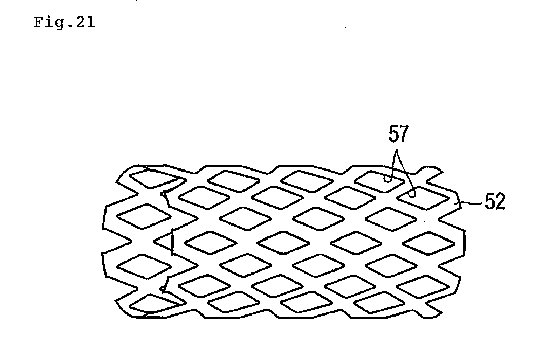

   FIG. 21 is a perspective view showing the condition where the biological adhesive tube shown in FIG. 20 is expanded.
[FIG. 22]

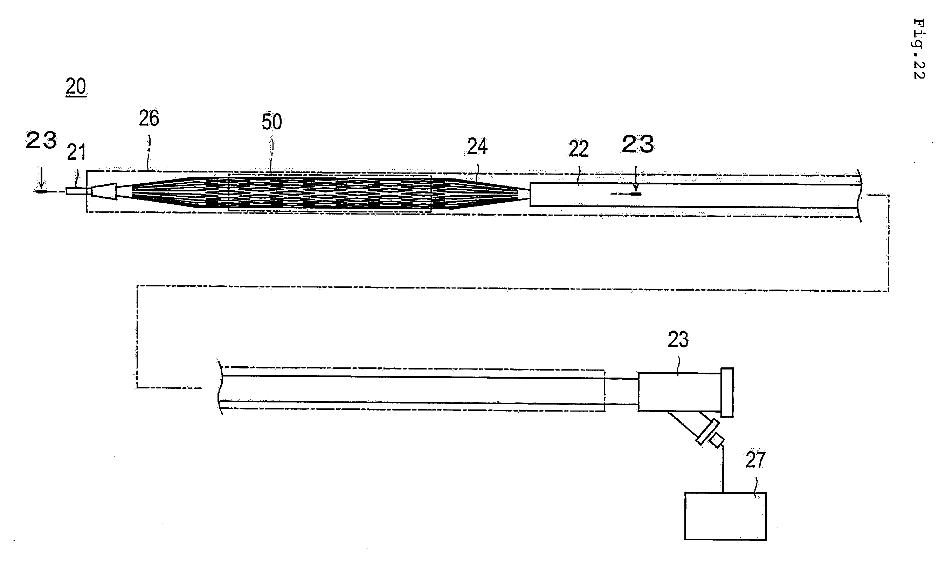

   FIG. 22 is a plan view showing a device for bonding a tube.
[FIG. 23]

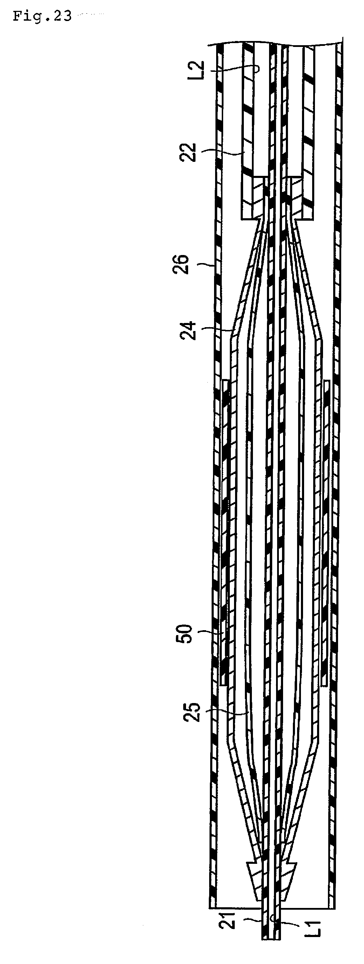

   FIG. 23 is a sectional view taken along line 23-23 of FIG. 22.
[FIG. 24]

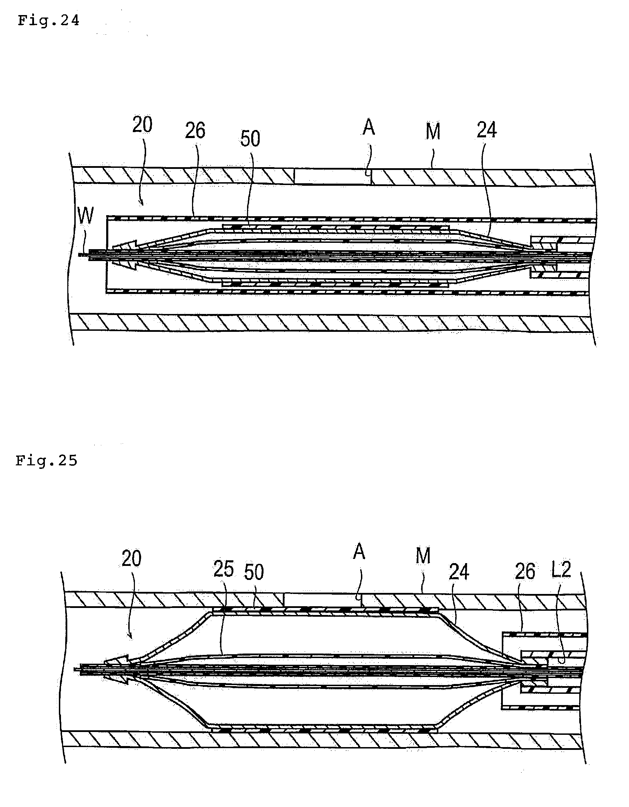

   FIG. 24 is a sectional view showing the condition where the device for bonding a tube is inserted in a blood vessel.
[FIG. 25]
   FIG. 25 is a sectional view showing the condition where the biological adhesive tube is tentatively fixed to a lesion by a holding section of the device for bonding a tube.
[FIG. 26]

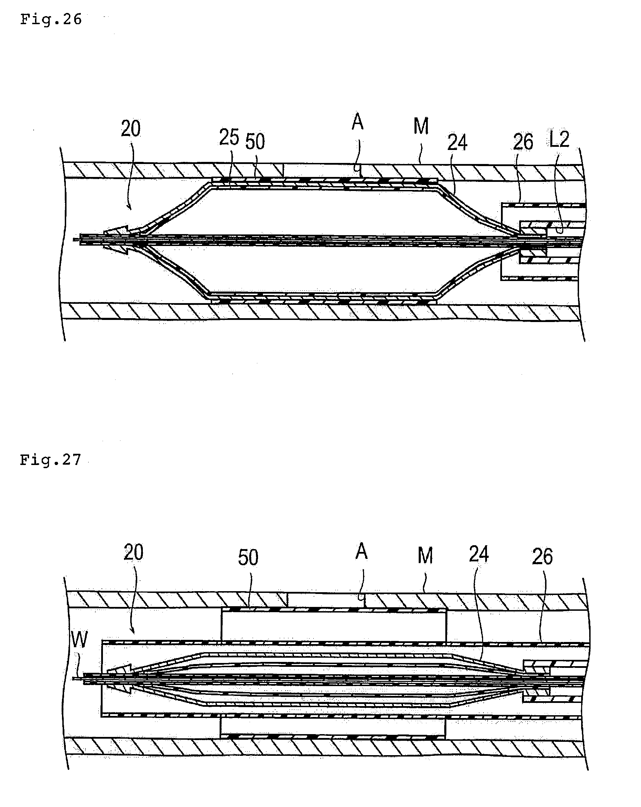

   FIG. 26 is a sectional view showing the condition where the biological adhesive tube is bonded to the lesion by a balloon of the device for bonding a tube.
[FIG. 27]
   FIG. 27 is a sectional view showing the condition where the holding section of the device for bonding a tube is contracted.
[FIG. 28]

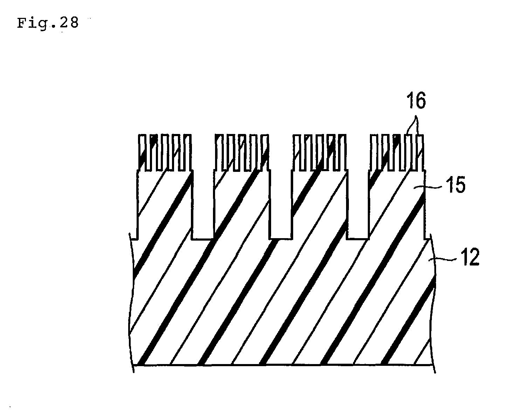

   FIG. 28 is a partly enlarged sectional view showing a further example of the biological adhesive sheet.

### Mode for Carrying Out the Invention

Now, embodiments of the present invention will be described below referring to the drawings. Incidentally, for convenience of description, the dimensional ratios in the drawings may be exaggerated so as to be different from actual ratios.

### <First Embodiment>

A biological adhesive sheet 10 according to a first embodiment of the present invention is a flexible medical sheet to be bonded to a living tissue M, and is one that exhibits an adhesive force both in gas and in liquid, without application of any separate adhesive to an adhesion surface. Besides, in the case where a stronger adhesive force is required, a stronger adhesive force can be obtained by a method in which a surface of the biological adhesive sheet 10 is made to be a more hydrophilic surface. The living tissue M to which the biological adhesive sheet 10 is to be bonded is not specifically restricted; for example, the living tissue M may be a lesion of a lumen such as blood vessel, bile duct, trachea, esophagus, urethra, etc. or the inside of nasal cavity, lung or the like. Particularly, examples of use of the biological adhesive sheet in a lumen include adhesion in the manner of covering a lesion for reinforcing or supplementing a weakened or defective blood vessel, heart or the like, adhesion for closing an entrance to an aneurysm or varix or the like, and adhesion for covering a vulnerable plaque formed in a blood vessel for the purpose of preventing lipid in the vulnerable plaque from flowing into the blood vessel.

The biological adhesive sheet 10 has a configuration as shown in FIGS. 1 to 3, wherein a plurality of fine projections 13 of the nanometer order are projectingly formed on one side of a flat plate-shaped substrate 12. In the condition where an adhesion surface 11 where the fine projections 13 are formed is in close contact with a living tissue M, the biological adhesive sheet 10 is pressed from the opposite side by a finger or other separate device, whereby the adhered state can be maintained by utilizing the van der Waals force between the fine projections 13 and the living tissue M, without use of any separate adhesive. Thus, the biological adhesive sheet 10 is configured so that the plurality of fine projections 13 are provided to increase the surface area of the adhesion surface 11, thereby generating a van der Waals force at such a magnitude that the adhered state of the sheet onto an object of adhesion can be maintained thereby. As a structure where adhesion is achieved by utilizing the van der Waals force, there is known, for example, the fine fibrous structure observed at the soles of feet of geckos.

The thickness B of the substrate 12 is preferably designed appropriately according to the application site of the living tissue M and the use of the biological adhesive sheet 10. For example, the thickness B is 3 to 3000 µm, preferably 30 to 300 µm.

The shape of the substrate 12 is not particularly restricted, and is preferably modified appropriately according to the shape of the lesion of the living tissue M to which the biological adhesive sheet 10 is to be applied. For example, the shape may be a circle, a rectangle or the like.

The size of the substrate 12 is not specifically restricted, and is preferably modified appropriately according to the width of the lesion of the living tissue M to which the biological adhesive sheet 10 is to be applied. For example, the maximum width C is 5 to 50 mm. The size of the substrate 12 can be selectively modified, for example, by preliminarily preparing a plurality of biological adhesive sheets 10 differing in size, or by cutting a large biological adhesive sheet 10 to an arbitrary size.

The projections 13 are formed in a columnar shape (in this embodiment, a cylindrical shape). The maximum outside diameter D of the projections 13 is 5 nm to 1 µm, more preferably 0.1 to 0.5 µm. The height H of the projections 13 is 1 to 500 µm, more preferably 10 to 50 µm. The pitch P of the projections 13 is 0 to 1 µm, more preferably 0.05 to 0.5 µm. Incidentally, the maximum outside diameter here means the length of a longest part in a section orthogonal to the extending direction (projecting direction) of the projection 13, and can be used even if the projections 13 are not circular in sectional shape.

The projections 13 are formed in a density of one or more projections per 1 µm², more preferably 50 or more projections per 1 µm². Where the projections 13 are shaped and sized as above-mentioned, an adhesive force can be exhibited based on the van der Waals force, both in gas and in liquid.

The pattern in which the projections 13 are arranged is not specifically restricted. Although the projections 13 are arranged regularly in this embodiment, they may be arranged irregularly.

While the projections 13 are formed to extend perpendicularly from the substrate 12 in this embodiment, they may be formed to be inclined relative to the substrate 12 as in another example shown in FIG. 4. The inclination angle X may be 0 to 60 degrees, preferably 0 to 30 degrees.

In addition, all the projections 13 may not necessarily be formed to extend in the same direction. For example, the projections 13 are inclined in different directions depending on the part in the adhesion surface 11 of the substrate 12.

Where the plurality of projections 13 are extended in the same direction (at the same inclination angle), upon adhesion to a living tissue M, the projections 13 tend to be aligned in one direction, as shown in FIG. 5. In this case, therefore, the biological adhesive sheet 10 can be easily peeled off by pulling it in one direction (see the arrow in FIG. 5), starting from one end of the substrate 12. Especially, since the biological adhesive sheet 10 according to this embodiment is adhered without use of any separate adhesive, it can be re-adhered after being peeled off. In addition, where the projections 13 are provided inclinedly so as to extend in the same direction as above-mentioned (see FIG. 4), the projections 13 can be made to be aligned in the same direction more easily upon adhesion, so that peeling thereof can be facilitated.

Besides, where the projections 13 are inclined in different directions depending on the part in the adhesion surface 11 of the substrate 12, the projections 13 are inclined in different directions depending on the part upon adhesion, making it difficult for the biological adhesive sheet 10 to peel off. This is effective in application of the biological adhesive sheet 10 to a part where inadvertent peeling is undesirable or a part where forces are exerted irregularly.

In addition, the shape of the projections 13 is not restricted to the cylindrical shape; for example, the shape may be a columnar shape with a polygonal section. Besides, the projection 13 may not necessarily have the same sectional shape from a proximal end portion thereof connected to the substrate 12 to a distal portion thereof. For instance, the projection 13 may be so formed that the section at its distal portion is greater or smaller than the section at its proximal portion.

The substrate 12 and the projections 13 are each formed of a biocompatible material. Preferably, at least one of them is formed of a biodegradable polymer. More preferably, both the substrate 12 and the projections 13 are formed of biodegradable polymers. In addition, at least one of the substrate 12 and the projections 13 may contain a biologically active agent, such as immunosuppressors and carcinostatic agents.

As the biodegradable polymer, for example, those which are high in stability in vivo are preferred. For instance, the biodegradable polymer is preferably at least one selected from the group consisting of polylactic acid, polylactic acid stereo complex, polyglycolic acid, a copolymer of polylactic acid and polyglycolic acid, polyhydroxybutyric acid, polymalic acid, poly-α-amino acid, collagen, laminin, heparan sulfate, fibronectin, vitronectin, chondroitin sulfate, hyaluronic acid, polycaprolactone, and polyamino acid. Taking into consideration its decomposition in vivo, the biodegradable polymer is preferably one that is safe medically. As the most preferred of these polymers, polylactic acid and its derivatives are used optimally.

The biocompatible material is not specifically restricted so long as it is biocompatible. Examples of other biocompatible materials than the above-mentioned biodegradable polymers include Teflon (registered trademark), polyurethane, and silicones.

The biologically active agent is not specifically restricted insofar as it is a substance that acts on the living tissue M. Especially where the biological adhesive sheet 10 is used to be set indwelling in a lumen, it is preferably one that has a restenosis-restraining effect or an inflammation-restraining effect. Examples of the biologically active agent include carcinostatic agent, immunosuppressor, antibiotic, antirheumatic, antithrombogenic agent, HMG-Co reductase inhibitor, ACE inhibitor, calcium antagonist, antilipemic agent, integrin inhibitor, antiallergic agent, antioxidant, GPIIbIIIa antagonist, retinoid, flavonoid, carotinoid, lipid improver, DNA synthesis inhibitor, tyrosine kinase inhibitor, antiplatelet agent, blood vessel smooth muscle proliferative inhibitor, antiflammatory agent, bio-derived material, interferon, and NO production promoting substance.

The carcinostatic agent is preferably, for example, vincristin, vinblastin, vindesin, irinotecan, pirarubicin, paclitaxel, docetaxel, methotrexate, or the like.

The immunosuppressor is preferably, for example, sirolimus, everolimus, pimecrolimus, sirolimus derivatives such as ABT-578, AP23573, CCI-779, etc., tacrolimus, azathioprine, cicrosporin, cyclophosphamide, mycophenolate mofetil, gusperimus, mizoribin, or the like.

The antibiotic is preferably, for example, mitomycin, adriamycin, doxorubicin, actinomycin, daunorubicin, idarubicin, pirarubicin, aclarubicin, epirubicin, peplomycin, zinostatin stimalamer, or the like.

The antirheumatic is preferably, for example, methotrexate, sodium thiomalate, penicillamine, lobenzarit, or the like.

The antithrombogenic agent is preferably, for example, heparin, aspirin, antithrombin preparation, ticlopidine, hirudin, or the like.

The HMG-CoA reductase inhibitor is preferably, for example, cerivastatin, cerivastatin sodium, atorvastatin, rosuvastatin, pitavastatin, fluvastatin, fluvastatin sodium, simvastatin, lovastatin, pravastatin, or the like.

The ACE inhibitor is preferably, for example, quinapril, perindopril erbumine, trandolapril, cilazapril, temocapril, delapril, enalapril maleate, lisinopril, captopril, or the like.

The calcium antagonist is preferably, for example, hifedipine, nilvadipine, diltiazem, benidipine, nisoldipine, or the like.

The antilipemic agent is preferably, for example, probucol.

The integrin inhibitor is preferably, for example, AJM300.

The antiallergic agent is preferably, for example, tranilast.

The antioxidant is preferably, for example, α-tocopherol.

The GPIIbIIIa antagonist is preferably, for example, abciximab.

The retinoid is preferably, for example, all-trans-retinoic acid.

The flavonoid is preferably, for example, epigallocatechin, anthocyanine, or proanthocyanidine.

The carotinoid is preferably, for example, β-carotene, or lycopene.

The lipid improver is preferably, for example, eicosapentanoic acid.

The DNA synthesis inhibitor is preferably, for example, 5-FU.

The tyrosine kinase inhibitor is preferably, for example, genistein, tyrphostin, erbstatin, staurosporine, or the like.

The antiplatelet agent is preferably, for example, ticlopidine, cilostazol, or clopidogrel.

The antiflammatory agent is preferably, for example, steroids such as dexamethasone, and prednisolone.

The bio-derived material is preferably, for example, EGF (epidermal growth factor), VEGF (vascular endothelial growth factor), HGF (hepatocyte growth factor), PDGF (platelet derived growth factor), BFGF (basic fibrolast growth factor), or the like.

The interferon is preferably, for example, interferon-γ1a.

The NO production promoting substance is preferably, for example, L-alginine.

Incidentally, whether the biologically active agent is to be one kind of biologically active agent or is to be a combination of two or more different kinds of biologically active agents should be appropriately selected according to the individual case.

The biological adhesive sheet 10 according to this embodiment is adhered (bonded) to a living organism by the van der Waals force by having the plurality of projections 13 in contact with the living organism, as above-mentioned. Therefore, other configuration for holding the adhered state is not needed, and it is possible to reduce the influence on the living organism and to enhance safety. Especially, in a therapy to the inside of a lumen wherein a stent is ordinarily necessary, the influence on healthy parts of the tube (vessel) can be reduced. Specifically, when a stent is used to the inside of a lumen, the stent makes contact with the whole body ranging over 360 degrees in the circumferential direction of the tube (lumen), even in the case where only a part in the circumferential direction of the tube is troubled; therefore, the stent is left indwelling in a wide range inclusive of healthy parts of the tube. When the biological adhesive sheet 10 is used, on the other hand, it can be adhered to a desired part by the adhesive force of the adhesion surface 11 itself, without utilizing radial forces as in the case of a stent. In this case, therefore, only the troubled part of the tube can be covered and treated, while minimizing the influence on the healthy parts of the tube.

Incidentally, though depending on the part to which the adhered biological adhesive sheet 10 is adhered, the biological adhesive sheet 10 thus adhered, for example, to the inside wall of a blood vessel, is embedded by being covered with the living tissue M with the lapse of time.

In addition, where the substrate 12 and the projections 13 are each formed of a biodegradable polymer, the adhered adhesive sheet 10 disappears by being decomposed with the lapse of time. In the case where the biological adhesive sheet 10 is adhered for example to the inside wall of a blood vessel, therefore, the period of medication with an antiplatelet agent for restraining generation of a thrombus due to the presence of the foreign matter can be shortened.

In addition, in the case where the substrate 12 and the projections 13 are both formed of biodegradable polymers such that the substrate 12 will disappear through decomposition earlier than the projections 13, the substrate 12 can be restrained from peeling before the adhesive force is lost by disappearance of the projections 13 through decomposition. The decomposition of the substrate 12 earlier than the projections 13 in this way can be realized, for example, by applying biodegradable polymers differing in decomposition time to the substrate 12 and the projections 13, respectively. For example, a configuration may be adopted wherein the projections 13 are formed of polycaprolactone or its derivative whereas the substrate 12 is formed of polylactic acid and its derivative.

Besides, as in a further example of the biological adhesive sheet shown in FIG. 6, the substrate 12 may be made to be a porous body by providing the substrate 12 with many through-holes 14. In this case, nutrients are supplied to the living tissues M through the through-holes 14 even after adhesion of the sheet to the living tissues M, whereby regeneration of the living tissues M is accelerated. Consequently, the biological adhesive sheet 10 can be more speedily covered with the living tissues M. The maximum outside diameter of the through-holes 14 formed in the substrate 12 is 0.1 to 100 µm, more desirably 0.5 to 20 µm.

In addition, where at least one of the substrate 12 and the projections 13 contains a biologically active agent, it is possible to treat a lesion to which the biological adhesive sheet 10 is applied, and to restrain obstruction or the like from occurring at the application site in the case where the biological adhesive sheet 10 is disposed to the inside of a body lumen.

Now, a device for bonding a sheet (sheet bonding device) 20 for delivering a patch to a lesion A of a blood vessel and adhere the patch to the lesion A, in using the biological adhesive sheet 10 according to this embodiment as the patch to be adhered so as to cover the lesion A, will be described below.

As shown in FIGS. 7 and 8, the sheet bonding device 20 includes: an elongate inner tube 21 formed therein with a guide wire lumen L1; an outer tube 22 disposed outside the inner tube 21; a hub 23 disposed at a proximal end of the outer tube 22; an annular holding section 24 which is disposed at a distal portion of the outer tube 22 and is self-expandable; an inflation balloon 25 disposed inside the holding section 24; and an outer sheath 26 externally covering the outer tube 22 and the holding section 24.

Between the outer tube 22 and the inner tube 21 is formed with a medium lumen L2 through which a fluid can flow for inflation/deflation of the balloon 25. The medium lumen L2 is in fluid-tight communication with the inside of the balloon 25.

The balloon 25 is so structured that it is deflated or folded on the outer circumference of the inner tube 21 when not inflated, and it is inflated when an inflation fluid is poured from an external fluid supply device 27 into the balloon 25 through the medium lumen L2.

Materials constituting the inner tube 21 and the outer tube 22 are each preferably a material having a certain degree of flexibility. As the materials, there can be used thermoplastic resins (which are ordinarily plastics), and thermosetting resins or thermo-crosslinkable resins such as rubber. Specific examples of the materials include: various thermoplastic resins and their polymeric derivatives, such as polyesters such as polyethylene terephthalate, polybutylene terephthalate, etc. and polyester elastomers using such polyesters as hard segments; polyolefins such as polyethylene, polypropylene, etc. and polyolefin elastomers; copolymeric polyolefins produced using a metallocene catalyst; vinyl polymers such as polyvinyl chloride, PVDC, PVDF, etc.; polyamides, inclusive of nylons, and polyamide elastomers (PAE); polyimides; polystyrene; SEBS resins; polyurethane; polyurethane elastomers; ABS resins; acrylic resins; polyarylates; polycarbonates; polyoxymethylene (POM); polyvinyl alcohol (PVA); fluororesins (ETFE, PFA, PTFE); ethylene-vinyl acetate saponified products; ethylene vinyl alcohol; ethylene vinyl acetate; carboxymethyl cellulose, methyl cellulose, cellulose acetate; vinyl polysulfones; liquid crystal polymers (LCP); polyether sulfones (PES); polyether ether ketones (PEEK); polyphenylene oxide (PPO); and polyphenylene sulfide (PPS). Other examples include thermosetting or crosslinkable resins such as vulcanized rubbers, silicone resins, epoxy resins, two-part reactive polyurethane resins, etc. Further, polymer alloys containing any of the above-mentioned thermoplastic resins and thermosetting or crosslinkable resins can also be used, and resin solutions prepared by dissolving these resins in solvent may also be used. The outside diameter of the outer tube 22 is 0.5 to 5 mm, preferably 1 to 3 mm.

Examples of the material which can be used to form the balloon 25 include ethylene-butylene-styrene block copolymers obtained by mixing polyethylene and ionomer with a low-molecular polystyrene and optionally with polypropylene; similar mixed materials obtained by replacing ethylene and butylenes in the above-mentioned polymers with butadiene or isoprene; polyvinyl chloride; polyurethane; (co)polyesters; polyamides and polyamide elastomers; thermoplastic rubbers; silicone-polycarbonate copolymer; and ethylene-vinyl acetate copolymers. The size of the balloon 25 depends on the part where the biological adhesive sheet 10 is to be used, and is not particularly limited. When inflated, the balloon 25 is preferably about 0.5 to 50 mm, more preferably about 1 to 5 mm.

The outer sheath 26 has such a structure that the outer tube 22 and the holding section 24 in a contracted state can be contained in the outer sheath 26. The outer sheath 26 is formed of a material which is low in the van der Waals force and which is difficult to adhere by the van der Waals force, in order that the biological adhesive sheet 10 held by the holding section 24 would not adhere to the outer sheath 26. The material is, for example, PTFE. The outside diameter of the outer sheath 26 is 1 to 10 mm, preferably 2 to 4 mm.

The holding section 24 is formed in a meshed tubular form from an elastic material. The holding section 24 is contained in the outer sheath 26 by being contracted elastically. The holding section 24 is elastically self-expandable so that when the outer sheath 26 is moved backward, the restraint on the holding section 24 by the outer sheath 26 is released. Incidentally, the structure of the holding section 24 may not necessarily be a meshed structure; the structure is not specifically restricted, insofar as the holding section 24 is self-expandable and can hold the biological adhesive sheet 10. The material of the holding section 24 is not particularly limited, so long as it can be elastically expanded and contracted. Examples of the material of the holding section 24 include stainless steel, and superelastic alloys (e.g., Ni-Ti alloy).

Now, a procedure for delivering the biological adhesive sheet 10 in a blood vessel and bonding it to a lesion A by the sheet bonding device 20 above-mentioned will be described below.

First, a plurality of biological adhesive sheets 10 are disposed on an outer circumferential surface of the radially contracted holding section 24 in an axially aligned state, with their adhesion surfaces on the outside. Thereafter, the holding section 24 is contained into the outer sheath 26, whereby the biological adhesive sheets 10 are stored between the holding section 24 and the outer sheath 26. In this instance, each of the biological adhesive sheets 10 is preferably held onto the holding section 24 by, for example, having a part thereof trapped in a gap in the meshed structure of the holding section 24. Alternatively, the biological adhesive sheets 10 may be adhered to the outer circumferential surface of the holding section 24 by an adhesive having a feeble adhesive force. Although the adhesion surface 11 of the biological adhesive sheet 10 makes contact with the inside surface of the outer sheath 26, the adhesion surface 11 of the biological adhesive sheet 10 is not adhered to the inside surface of the outer sheath 26, since the outer sheath 26 is made of PTFE. Incidentally, while three biological adhesive sheets 10 are arrayed on the outer circumferential surface of the holding section 24 in the drawings, the number of the biological adhesive sheets 10 may be two or less, or may be four or more.

Next, a blood vessel at the patient's leg or arm is minutely incised, an introducer sheath is set there, and a guide wire W is precedingly advanced to a target position through a lumen of the introducer sheath under radioscopy. Then, the guide wire W is inserted and passed in the guide wire lumen L1 of the sheet bonding device 20, and the sheet bonding device 20 is moved along the guide wire W. In this instance, the biological adhesive sheets 10 are delivered while being contained between the holding section 24 and the outer sheath 26. Then, after the biological adhesive sheet 10 on the most distal side of the plurality of biological adhesive sheets 10 held on the holding section 24 is delivered into the vicinity of the lesion A of a blood vessel, as shown in FIG. 9, the insertion of the sheet bonding device 20 is stopped. Thereafter, the outer sheath 26 is retracted by pulling on the proximal side until the biological adhesive sheet 10 on the most distal side of the plurality of biological adhesive sheets 10 held on the holding section 24 is exposed into the blood vessel, as shown in FIG. 10. This ensures that a distal side of the holding section 24 released from the restraint by the outer sheath 26 expands by its self-expanding function, whereby the biological adhesive sheet 10 held on the outer circumferential surface of the holding section 24 is tentatively fixed so as to cover the lesion A of the blood vessel. Thereafter, the inflation fluid is poured from the fluid supply device 27 (see FIG. 7) into the balloon 25 through the medium lumen L2, thereby inflating the balloon 25, as shown in FIG. 11. This ensures that the biological adhesive sheet 10 so arranged as to make contact with the lesion A at its adhesion surface 11 by the holding section 24 is pressed against the lesion A with pressing force stronger than that exerted by the holding section 24, so that the biological adhesive sheet 10 is adhered to the blood vessel wall by the van der Waals force in such a manner as to cover the lesion A. Incidentally, in the case where a stent is set indwelling in a blood vessel, the pressure inside the balloon is ordinarily about 8 to 10 atm. On the other hand, for adhesion of the biological adhesive sheet 10 according to this embodiment, a pressure of about 1 to 2 atm is sufficient for adhesion, so that the blood vessel can be restrained from being over-inflated.

Next, the inflation fluid is discharged from the inside of the balloon 25 through the medium lumen L2 by the fluid supply device 27, thereby deflating the balloon 25. Then, as shown in FIG. 12, the outer sheath 26 is advanced, to contain the holding section 24 into the outer sheath 26 while radially contracting the holding section 24. Thereafter, the guide wire W is moved to another lesion A, and the sheet bonding device 20 is moved along the guide wire W, whereby the biological adhesive sheet 10 on the most distal side of the plurality of biological adhesive sheets 10 left on the holding section 24 is delivered into the vicinity of the another lesion A. This is followed by stopping the insertion of the sheet bonding device 20. Then, in the same manner as the procedure for bonding the first biological adhesive sheet 10, the outer sheath 26 is retracted to tentatively fix the second biological adhesive sheet 10 by the holding section 24, and the second biological adhesive sheet 10 is pressed against and adhered to the blood vessel wall by the balloon 25. Thereafter, the balloon 25 is again deflated, and the outer sheath 26 is advanced to contain the holding section 24 into the outer sheath 26 while radially contracting the holding section 24. The same procedure as above is repeated, whereby the biological adhesive sheets 10 are sequentially adhered to a plurality of lesions A of the blood vessel. After the treatments of all the lesions A are completed or after all the plurality of biological adhesive sheets 10 held on the holding section 24 are used up, the sheet bonding device 20 is removed, with the holding section 24 contained in the outer sheath 26, to complete the procedure.

According to the sheet bonding device 20 in this embodiment, the expandable and contractible holding section 24 with the biological adhesive sheets 10 held on the outer circumferential surface thereof is contained in a contracted state in the outer sheath 26. Therefore, the biological adhesive sheets 10 can be easily delivered to target positions. Furthermore, by moving the outer sheath 26 in the axial direction, the holding section 24 is permitted to expand by its self-expanding function. Therefore, the biological adhesive sheets 10 held on the outer circumferential surface of the holding section 24 can be easily adhered to the living organism by operating the device on the proximal side. In addition, since the balloon 25 is provided, the biological adhesive sheets 10 can be adhered to the living organism more assuredly by operations on the proximal side.

Besides, the balloon 25 is capable of inflation and deflation inside the holding section 24. This ensures that, with the biological adhesive sheet 10 tentatively fixed to the living tissue M by the holding section 24, the biological adhesive sheet 10 can be pressed by the balloon 25, to be assuredly adhered to the living tissue M. Incidentally, the balloon 25 may not necessarily be capable of inflation and deflation inside the holding section 24. For example, a configuration can be adopted wherein a balloon is disposed inside a holding section so that it can be moved forward and backward in the axial direction, and the balloon can be inflated and deflated in the state of being exposed from the outer sheath by moving it to the distal side relative to the holding section. In addition, if the biological adhesive sheet 10 can be firmly adhered to the living tissue M by only the holding section 24, a configuration may be adopted in which only the holding section 24 is provided without providing any balloon 25. Besides, a configuration may be adopted in which the holding section 24 is not provided, and the biological adhesive sheet 10 is adhered while being delivered by the balloon 25 alone.

Now, an example of the method of manufacturing the biological adhesive sheet 10 will be described below.

First, a polymethyl methacrylate resin (PMMA) supported on a silicon wafer is formed with a fine pattern 31 of pores of the order of several hundreds of nanometers by electron beam lithography, to produce a mold 30 (see FIG. 13). The shape of the fine pattern 31 is determined so as to correspond to the shape obtained when the projections 13 of the adhesion surface 11 of the biological adhesive sheet 10 to be produced are transferred.

Next, the above-mentioned biodegradable polymer (or biocompatible material) as the material for the substrate 12 and the projections 13 is dissolved in a solvent in a concentration of 0.001 to 1 wt%, to obtain a sol phase. As the solvent, chloroform or the like can be applied.

Subsequently, the mold 30 is held horizontal so that its surface formed with the fine pattern 31 is oriented upward. The material in the sol phase is poured into the mold 30 so that the material enters into the fine pattern 31, and, further, the material in an amount corresponding to the thickness B of the substrate 12 is poured as shown in FIG. 14. Thereafter, the mold 30 is heated to a temperature in the range from room temperature to 40 degrees, thereby evaporating off the solvent and solidifying the material. Incidentally, in the case of applying different materials for the substrate 12 and the projections 13, a production method may be adopted wherein the material in a sol phase is poured into the mold 30, to permit the material to enter into the fine pattern 31, and thereafter a different material dissolved in a solvent is poured in an amount corresponding to a predetermined thickness. In addition, where the material is thermoplastic, a method may be adopted wherein the material is melted by heating, the melt is poured into the mold 30, and is cooled to solidify.

After the material(s) is solidified, the solidified material(s) is detached from the mold 30, to obtain a biological adhesive sheet 10 having a plurality of projections 13 formed on a substrate 12.

Incidentally, the method for processing the pattern of the order of several hundreds of nanometers is not restricted to the above-mentioned method. For example, nanoimprint, soft lithography, shaping by use of a fine cutting tool (e.g., diamond cutting tool), and the like can also be applied. The method is preferably selected appropriately according to the condition of the biological adhesive sheet 10 such as shape, dimension, material and the like.

### <Second Embodiment>

A biological adhesive tube 50 (biological adhesive sheet) according to a second embodiment of the present invention is a medical tubular body to be adhered particularly to the inside wall surface of a lumen of a living tissue M. The biological adhesive tube 50 exhibits an adhesive force both in gas and in liquid, without application of any separate adhesive to an adhesion surface. Like in the first embodiment, the living tissue M to which the biological adhesive tube 50 is to be adhered is not specifically restricted.

As shown in FIGS. 16 to 18, the biological adhesive tube 50 has a configuration in which a plurality of fine projections 53 of the order of nanometers are projectingly formed at an outer circumferential surface of a tubular base (or tubular body) 52 (substrate). When the adhesion surface 51 (outer circumferential surface) where the fine projections 53 are formed is put in close contact with a living tissue M and the biological adhesive tube 50 is pressed from the opposite side by a separate device or the like, the adhered state can be maintained by utilizing the van der Waals force between the fine projections 53 and the living tissue M, without use of any separate adhesive. Specifically, the biological adhesive tube 50 has a structure in which the plurality of fine projections 53 are provided to increase the surface area of the adhesion surface 51, thereby generating a van der Waals force at such a magnitude that the adhered state of the adhesion surface 51 onto an object of adhesion can be maintained.

The thickness B of the base 52 is preferably designed appropriately according to the application site of the living tissue M and the use of the biological adhesive tube 50. For example, the thickness B is 3 to 3000 µm, more preferably 30 to 300 µm.

The axial length L of the base 52 is not particularly limited, and is preferably modified appropriately depending on the width of a lesion of the living tissue M to which the biological adhesive tube 50 is to be applied. For example, the axial length L is 5 to 50 mm.

The outside diameter G of the base 52 is not specifically restricted, and is preferably modified appropriately according to the width of the lesion of the living tissue M to which the biological adhesive tube 50 is to be applied. For example, the outside diameter G is 1 to 5 mm.

The axial length L and the outside diameter G of the base 52 can be selectively modified, for example, by preliminarily producing a plurality of biological adhesive tubes 50 differing in size, or can be arbitrarily modified by cutting a large biological adhesive tube 50 to an arbitrary size. The material for the base 52 is the same as the material for the substrate 12 in the first embodiment, and, therefore, description thereof is omitted here for avoiding redundancy.

The projections 53 are formed in a columnar shape (in this embodiment, a cylindrical shape). Incidentally, the configuration of the projections 53 provided on the base 52 is the same as the projections 13 provided on the substrate 12 in the first embodiment, and, therefore, description thereof is omitted here for avoiding redundancy.

In the biological adhesive tube 50 according to the second embodiment, adhesion is achieved by the van der Waals force generated when the plurality of projections 53 are put into contact with a living organism, as above-mentioned. Therefore, other configuration for holding an adhered state is unnecessary, so that influence on the living organism can be reduced and safety can be enhanced. Especially, in a therapy to the inside of a lumen wherein a stent (which is a strength supporting material) is ordinarily necessary, the influence on healthy parts of the tube can be reduced. Specifically, when a stent is used to the inside of a lumen, the lumen receives radial forces in a radial direction from the stent. When the biological adhesive tube 50 is used, on the other hand, adhesion can be achieved by the adhesive force of the adhesion surface 51 itself, without utilizing any radial forces, so that the influence on the lumen can be minimized as much as possible.

Incidentally, though depending on the part to which the biological adhesive tube 50 is adhered, the adhered biological adhesive tube 50 thus adhered, for example, to the inside wall of a blood vessel, is embedded by being covered with the living tissue M with the lapse of time.

In addition, where the base 52 and the projections 53 are each formed of a biodegradable polymer, the adhered biological adhesive tube 50 disappears by being decomposed with the lapse of time. In the case where the biological adhesive tube 50 is adhered to the inside wall of a blood vessel, therefore, the period of medication with an antiplatelet agent for restraining generation of a thrombus due to the presence of the foreign matter can be shortened.

Besides, in the case where the base 52 and the projections 53 are both formed of biodegradable polymers such that the base 52 will disappear through decomposition earlier than the projections 53, the base 52 can be restrained from peeling before the adhesive force is lost by disappearance of the projections 53 through decomposition. The decomposition of the base 52 earlier than the projections 53 in this way can be realized, for example, by applying biodegradable polymers differing in decomposition time to the base 52 and the projections 53, respectively. For example, a configuration may be adopted wherein the projections 53 are formed of polycacrolactone and its derivative whereas the base 52 is formed of polylactic acid and its derivative.

In addition, as in another example of the biological adhesive tube shown in FIG. 19, the base 52 may be made to be a porous body by providing the base 52 with many through-holes 54. In this case, nutrients are supplied to the living tissues M through the through-holes 54 even after adhesion of the adhesive tube to the living tissues M, whereby regeneration of the living tissues M is accelerated. As a result, the biological adhesive tube 50 can be more speedily covered with the living tissues M. The maximum outside diameter of the through-holes 54 formed in the base 52 is 0.1 to 100 µm, more desirably 0.5 to 20 µm.

Besides, where at least one of the base 52 and the projections 53 contains a biologically active agent, it is possible to treat a lesion to which the biological adhesive tube 50 is applied, and to restrain obstruction or the like from occurring at the application site in the case where the biological adhesive tube 50 is disposed to the inside of a body lumen.

In addition, as in a further example of the biological adhesive tube shown in FIG. 20, a configuration can be adopted wherein the base 52 is provided with a plurality of axially extending slits 57 so that the base 52 can be expanded while deforming radially, as shown in FIG. 21. The slits 57 may be formed preliminarily in a penetrating form, or may be formed as grooves having such a depth that they do not penetrate until receiving a expanding pressure from the inside. Besides, the slits 57 may be formed in their intermediate portions with non-cut parts which hold the shape before expanding; in this case, the non-cut parts are cut apart by the expanding pressure exerted from the inside, thereby spreading the slits 57.

Now, a procedure for delivering the biological adhesive tube 50 in a blood vessel and bonding it to a lesion A, by use of the tube bonding device 20 described in the first embodiment, will be described below.

First, as shown in FIGS. 22 and 23, the biological adhesive tube 50 is disposed on the outer circumferential surface of a radially contracted holding section 24, with the adhesion surface 51 on the outside, after which the holding section 24 is contained into the outer sheath 26, and the biological adhesive tube 50 is contained between the holding section 24 and the outer sheath 26. In this instance, the biological adhesive tube 50 is in the state of being smaller than its practical diameter (size) by being folded, for example. In addition, the biological adhesive tube 50 is preferably held onto the holding section 24 by, for example, having a part thereof trapped in a gap in the meshed structure of the holding section 24. Alternatively, the biological adhesive tube 50 may be adhered to the outer circumferential surface of the holding section 24 by an adhesive having a feeble adhesive force. Although the adhesion surface 51 of the biological adhesive tube 50 makes contact with the inside surface of the outer sheath 26, the adhesion surface 51 of the biological adhesive tube 50 is not adhered to the inside surface of the outer sheath 26, since the outer sheath 26 is made of PTFE.

Next, a blood vessel at the patient's leg or arm is minutely incised, an introducer sheath is set there, and a guide wire W is precedingly advanced to a target position through a lumen of the introducer sheath under radioscopy. Then, the guide wire W is inserted and passed in a guide wire lumen L1 of a tube bonding device 20, and the tube bonding device 20 is moved along the guide wire W. In this instance, the biological adhesive tube 50 is delivered while being contained between the holding section 24 and the outer sheath 26. Then, after the biological adhesive tube 50 held on the holding section 24 is delivered into the vicinity of lesion A of a blood vessel, as shown in FIG. 24, the insertion of the tube bonding device 20 is stopped. Thereafter, the outer sheath 26 is retracted by pulling on the proximal side until the biological adhesive tube 50 held on the holding section 24 is exposed into the blood vessel, as shown in FIG. 25. This ensures that the holding section 24 released from the restraint by the outer sheath 26 expands by its self-expanding function, whereby the biological adhesive tube 50 held on the outer circumferential surface of the holding section 24 is tentatively fixed so as to cover the lesion A of the blood vessel. Thereafter, an inflation fluid is poured from a fluid supply device 27 (see FIG. 22) into the balloon 25 through the medium lumen L2, thereby inflating the balloon 25, as shown in FIG. 26. This ensures that the biological adhesive tube 50 so arranged as to make contact with the lesion A at its adhesion surface 51 by the holding section 24 is pressed against the lesion A with pressing force stronger than that exerted by the holding section 24, so that the biological adhesive tube 50 is adhered (bonded) to the blood vessel wall by the van der Waals force in such a manner as to cover the lesion A. Incidentally, in the case where a stent is set indwelling in a blood vessel, the pressure inside the balloon is ordinarily about 8 to 10 atm. On the other hand, for adhesion of the biological adhesive tube 50 according to the this embodiment, a pressure of about 1 to 2 atm is sufficient for adhesion, so that the blood vessel can be restrained from being over-inflated.

Next, the inflation fluid is discharged from the inside of the balloon 25 through the medium lumen L2 by the fluid supply device 27, thereby deflating the balloon 25. Then, as shown in FIG. 27, the outer sheath 26 is advanced, to contain the holding section 24 into the outer sheath 26 while radially contracting the holding section 24. Thereafter, the tube bonding device 20 is removed from the blood vessel, to complete the procedure.

Incidentally, in this embodiment, only one biological adhesive tube 50 is disposed on the holding section 24. However, a configuration may be adopted wherein a plurality of biological adhesive tubes 50 are disposed so as to be axially arrayed on the outer circumferential surface of the holding section 24, and are exposed into the blood vessel one by one by the outer sheath 26, whereby the plurality of biological adhesive tubes 50 are sequentially set indwelling at a plurality of lesions A in the blood vessel.

Now, an example of the method of manufacturing the biological adhesive tube 50 will be described below.

First, in the same manner as the procedure described in the first embodiment, a flat plate-shaped sheet formed by solidification of a material is prepared by use of a mold 30. Incidentally, description of the procedure for preparing the sheet is omitted here for avoiding redundancy.

Thereafter, the sheet is cut to a predetermined size, after which the cut piece is curved into a tubular shape so that the projections 53 are located on the outside, and the overlapping portions are bonded to each other by crimping or by use of an adhesive composed of a biodegradable polymer (or a biocompatible material). In this manner, a biological adhesive tube 50 is obtained wherein a plurality of projections 53 are formed on an outer circumferential surface of a base 52.

The present invention is not restricted only to the above-described embodiments, and various modifications can be made within the technical thought of the invention by those skilled in the art. For instance, the shape of the projections is not limited to the above-mentioned form, insofar as the projections can be adhered (bonded) by the van der Waals force. As shown in FIG. 28, the projections may have a form wherein a plurality of projections 16 are formed to project from each of a plurality of projected parts 15 formed on a substrate 12. In addition, the projections may each be conical or pyramidal in shape. Where the projections are pyramidal in shape, they can be easily produced by forming grooves lengthwise and crosswise by use of a minute cutting tool.

In addition, the projections may be formed on both sides of the substrate (base). Besides, the substrate (base) may be formed in a tubular shape, and the projections may be formed on the inside surface or the outside surface of the substrate (base).

In addition, the biological adhesive sheet 10 according to the first embodiment may be provided with slits, like in another example in the second embodiment shown in FIGS. 20 and 21.

Furthermore, the present application is based on Japanese Patent Application No. 2010-212795 and Japanese Patent Application No. 2010-212797 filed on September 22, 2010, the contents of disclosure of which are entirely incorporated herein by reference.

### Explanation of Reference Symbols

- 10: Biological adhesive sheet
- 11, 51: Adhesion surface
- 12: Substrate
- 13, 16, 53: Projection
- 14, 54: Through-hole
- 20: Device for bonding sheet
- 21: Inner tube
- 22: Outer tube
- 24: Holding section
- 25: Balloon
- 26: Outer sheath
- 50: Biological adhesive tube (biological adhesive sheet)
- 52: Base (substrate)
- 57: Slit
- B: Thickness of substrate
- D: Maximum outside diameter of projection
- H: Height of projection

## Claims

1. A biological adhesive sheet comprising:
a substrate formed of a biocompatible material; and
a plurality of projections which are formed of a biocompatible material and are projected from a surface of the substrate,
wherein the biological adhesive sheet is bonded to a living tissue by van der Waals forces by having the plurality of projections in contact with the living tissue.

2. The biological adhesive sheet according to claim 1, wherein the projections are formed in a density of one or more projections per 1 µm² on the surface of the substrate, and each have a length of 1 to 500 µm and a maximum outside diameter of 5 nm to 1 µm.

3. The biological adhesive sheet according to claim 1 or 2, wherein the substrate and the projections are each formed of a biodegradable polymer.

4. The biological adhesive sheet according to claim 3, wherein the substrate and the projections are each formed of biodegradable polymer such that the substrate disappears through decomposition earlier than the projections.

5. The biological adhesive sheet according to any one of claims 1 to 4, wherein the biodegradable polymer is one or more selected from the group consisting of polylactic acid, polylactic acid stereo complex, polyglycolic acid, a copolymer of polylactic acid and polyglycolic acid, polyhydroxybutyric acid, polymalic acid, poly-α-amino acid, collagen, laminin, heparan sulfate, fibronectin, vitronectin, chondroitin sulfate, hyaluronic acid, polycaprolactone, and polyamino acid.

6. The biological adhesive sheet according to any one of claims 1 to 5, wherein the substrate is a porous body.

7. The biological adhesive sheet according to any one of claims 1 to 6, wherein at least one of the substrate and the projections contains a biologically active agent.

8. The biological adhesive sheet according to any one of claims 1 to 7, wherein the substrate is a tubular base, and the projections are formed to project from an outer circumferential surface of the base and are formed in a tubular shape.

9. The biological adhesive sheet according to any one of claims 1 to 8, wherein the substrate has a plurality of slits.

10. A device for bonding a sheet, comprising:
a tube which can be inserted into a living organism;
a tubular holding section which is disposed at a distal portion of the tube, is cable of being expanded and contracted elastically, and is capable of holding on an outer circumferential surface thereof a biological adhesive sheet to be bonded to a living tissue;
an outer sheath which contracts the holding section by covering the outer circumference of the holding section, and permits releases and expansion of the holding section by coming away from the holding section along an axial direction; and
a balloon which is disposed at a distal portion of the tube, and is capable of being inflated and deflated.

11. The device for bonding a sheet according to claim 10, wherein the balloon is capable of being inflated and deflated inside the holding section.
